(19) Europäisches Patentamt
European Patent Office
Office européen des brevets

(11) EP 2 417 966 A1

(12) EUROPEAN PATENT APPLICATION
published in accordance with Art. 153(4) EPC

(43) Date of publication:
**15.02.2012 Bulletin 2012/07**

(21) Application number: **10761649.2**

(22) Date of filing: **02.04.2010**

(51) Int Cl.:
***A61K 8/81*** (2006.01) ***A61K 8/34*** (2006.01)
***A61K 8/91*** (2006.01) ***A61Q 5/06*** (2006.01)

(86) International application number:
**PCT/JP2010/056057**

(87) International publication number:
**WO 2010/116950 (14.10.2010 Gazette 2010/41)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.04.2009 JP 2009092516**

(71) Applicant: **Shiseido Company, Ltd.**
**Chuo-ku**
**Tokyo 104-8010 (JP)**

(72) Inventors:
- **TOYODA, Tomonori**
**Yokohama-shi**
**Kanagawa 224-8558 (JP)**
- **SHIMIZU, Hideki**
**Yokohama-shi**
**Kanagawa 224-8558 (JP)**
- **KURASHIMA, Takumi**
**Yokohama-shi**
**Kanagawa 224-8558 (JP)**
- **FUJIYAMA, Taizo**
**Yokohama-shi**
**Kanagawa 224-8558 (JP)**
- **MIYAZAWA, Kazuyuki**
**Yokohama-shi**
**Kanagawa 224-8558 (JP)**

(74) Representative: **Henkel, Breuer & Partner**
**Patentanwälte**
**Maximiliansplatz 21**
**80333 München (DE)**

# (54) HAIR COSMETIC

(57) Disclosed is a hair cosmetic which can achieve fixing of hair (hairstyle) by sticking and also has an excellent arranging ability (also including a restyling ability), and moreover has humidity resistance. Specifically disclosed is a hair cosmetic comprising a novel adhesive setting resin which is obtained by combining and polymerizing monomers having a specific structure and has a moderate firmness and a high adhesive force when forming a film and further comprising a setting agent resin. Accordingly, a hair cosmetic having both fixing ability and arranging ability, and also having excellent humidity resistance (hair set retaining ability) is obtained.



Printed by Jouve, 75001 PARIS (FR)

## Description

Technical Field

The present invention relates to a hair cosmetic that has both the abilities to set and arrange hair (also including the ability to restyle hair) and is further excellent in moisture resistance. More specifically, the present invention relates to a hair cosmetic that is capable of setting hair based on fixation and restyling the hair and is excellent in the ability to retain set hairstyles, by virtue of a novel polymer contained therein and a setting resin further incorporated therein.

Background Art

Hair styling includes two functions: forming hairstyles and keeping the formed hairstyles. The principles on which these two functions are exerted are allegedly fixation and adhesion (Non-Patent Document 1).

Hair styling based on fixation is to set hair by forming a solid film using a film-forming agent (polymer resin) called a setting agent. For example, conventional hair gels or hair sprays are based mainly on a hair styling mechanism using a setting resin. For example, Patent Document 1 discloses a hair cosmetic mainly comprising a film-forming polymer such as polyvinylpyrrolidone, sodium polyacrylate, or a polyvinylpyrrolidone-polyvinyl acetate copolymer as a setting resin. Patent Document 2 discloses a hair cosmetic comprising a silylated urethane resin as a setting resin, and has reported that this hair cosmetic forms a film having both softness and hardness and has natural textures and a high ability to keep hairstyles.

However, the hair cosmetics obtained using the resins as described in Patent Documents 1 and 2 disadvantageously fails to restyle hair from a temporarily formed hairstyle due to a hard film formed by the setting resin and loses styling functions when the film is broken. Specifically, a problem of styling agents based on fixation brought about by such a setting resin is the poor ability to arrange hair, though they are excellent in setting hair.

On the other hand, styling based on adhesion is to allow hairs to adhere to one another by an oily ingredient. Hair liquids comprising an adhesive oily ingredient such as polyalkylene glycol as a main base, hair waxes that utilize the adhesiveness of a solid oil and have been preferred by the youth in recent years, and so on are known as such styling agents. For example, Patent Document 3 discloses a cosmetic for hair that comprises waxes and a spinnable water-soluble polymer and is excellent in restyling hair.

However, hair styling based on the adhesiveness of such an oily ingredient is characterized by being capable of restyling through fingers or a brush because the oily ingredient retains flowability and adhesiveness on the hair. A problem of this hair styling is that the ability to set hair (ability to keep hairstyles) as in hair cosmetics comprising a setting resin is not obtained, though it is excellent in the so-called ability to arrange hair.

Prior Art Documents

Non-Patent Documents

Non-Patent Document 1: "Development of Advanced Cosmetics II", ed. by Masato Suzuki, published by CMC Publishing Co., Ltd., 1996, Chapter 10: Functions of Hair-Styling Agents and State-of-the-Art Technology

Patent Documents

Patent Document 1: JP-A-2006-213706
Patent Document 2: JP-A-2003-171244
Patent Document 3: JP-A-Hei 10-45546

Summary of the Invention

Problems to be solved by the Invention

Accordingly, an object of the present invention is to provide a hair cosmetic that is capable of setting hair (hairstyle) based on fixation, is also excellent in the ability to arrange hair (also including the ability to restyle hair), and further has the ability to retain set hairstyles enhanced by improving moisture resistance.
The present inventors have conducted diligent studies to attain the object and consequently completed the present invention by finding that: a hair cosmetic having both the abilities to set and arrange hair is obtained by incorporating,

as a setting resin, a novel adhesive setting resin having moderate hardness and high adhesive strength during film formation; and moisture resistance (the ability to retain set hairstyles) can be improved by further incorporating therein a setting agent resin.

Means for Solving the Problems

Specifically, the present invention provides a hair cosmetic comprising an adhesive setting resin, a setting agent resin, and an alcohol, wherein the adhesive setting resin being obtained by polymerizing:

at least one monomer represented by the following formula (A) (hereinafter, referred to as a "monomer A"):

[Formula 1]

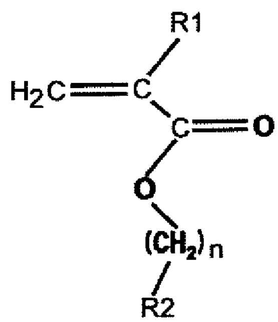

(A)

wherein R1 represents H or $CH_3$; n represents an integer of 0 to 30; $(CH_2)_n$ contains a branched chain; and R2 represents H, OH, $OCH_3$, $OCH_2CH_3$, or phenyl,
and/or
at least one monomer represented by the following formula (B) (hereinafter, referred to as a "monomer B"):

[Formula 2]

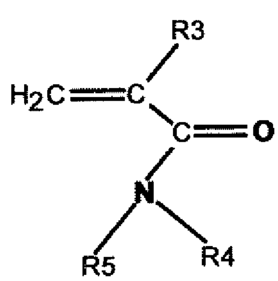

(B)

wherein R3 represents H or $CH_3$; R4 and R5, which may be the same or different, each represent H or $(CH_2)_1R'$; 1 represents an integer of 1 to 3; R' represents H, OH, or -NR"R"'; and R" and R"', which may be the same or different, each represent H or a C1-C3 alkyl group,
and
at least one monomer represented by the following formula (C) (hereinafter, referred to as a "monomer C"):

[Formula 3]

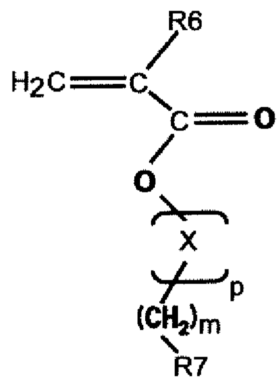

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or glyceryl,
and
at least one monomer represented by the following formula D (hereinafter, referred to as a "monomer D"):

[Formula 4]

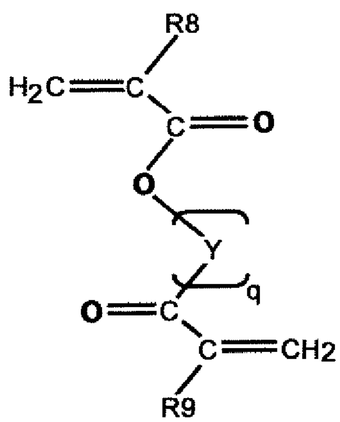

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or a glyceryl group, provided that q represents 1 when Y represents the oxyalkylene group having 5 or more carbon atoms.

Effects of the Invention

A hair cosmetic of the present invention can have both the abilities to set and arrange hair, which are impossible to achieve for conventional setting resins, by incorporating therein the novel adhesive setting resin described above. Furthermore, the hair cosmetic of the present invention can be excellent in moisture resistance and have the improved ability to retain set hairstyles for a long time, by further incorporating therein a setting agent resin conventionally used, in addition to the adhesive setting resin.

Modes for Carrying out the Invention

A hair cosmetic of the present invention comprises, as an essential ingredient, an adhesive setting resin obtained

by polymerizing the monomers A and/or B, and C, and D described above. Specifically, for the adhesive setting resin of the present invention, it is essential to comprise the monomers C and D. The adhesive setting resin of the present invention can comprise any one of the monomers A and B, or both. A resin (polymer) that lacks the monomer C or D produces neither favorable adhesive strength nor the ability to arrange hair (ability to restyle hair).

It is particularly preferred that the adhesive setting resin used in the present invention should have a structure represented by the following formula (I):

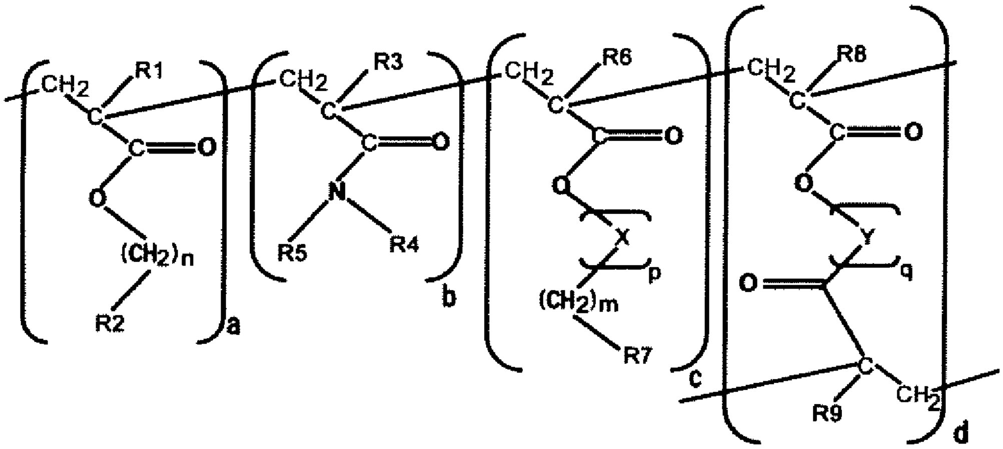

(I)

In the formula (I), R1 to R9, n, m, p, and q are as defined in the formulas A to D. The terms in the present specification are used as usual meanings. For example, an oxyethylene group (EO), an oxypropylene group (PO), and an oxybutylene group (BO) mean linear or branched oxyalkylene groups having 2, 3, and 4 carbon atoms, respectively. Moreover, in the formula (I), a represents a number within the range of $40 < a < 400$; b represents a number within the range of $80 \leq b < 300$; c represents a number within the range of 30 < c 300; and d represents a number within the range of $0 < d < 10$. The percentage by mass of each monomer in the adhesive setting resin (polymer of the formula (I)) that satisfies the conditions described above is approximately as follows: $7.5 < A < 62.5$, $20 \leq B < 45$, $7.5 < C < 60$, and $0 < D < 5$.

The adhesive setting resin of the present invention can be prepared by mixing the monomers A and/or B, and C, and D at an appropriate ratio and polymerizing the mixture through reaction using a standard method, if necessary, in an appropriate solvent. For example, the adhesive setting resin can be obtained by thermally polymerizing the mixture at approximately 80˚C for 8 hours using a polymerization initiator such as 2,2'-azobisisobutyronitrile in ethanol. The obtained polymer can be purified appropriately for use.

The amount of the adhesive setting resin incorporated in the hair cosmetic of the present invention can vary depending on the product form thereof and is generally 0.1 to 30% by mass, preferably 1 to 20% by mass, more preferably 2 to 15% by mass. If the amount is less than 0.1% by mass, sufficient effect may not be exerted even in combination with a setting resin. If the adhesive setting resin is incorporated in an amount exceeding 30% by mass, the resulting hair cosmetic may make hair bristly.

The hair cosmetic of the present invention comprises a setting agent resin in addition to the adhesive setting resin. The setting agent resin in the present invention is an anionic, cationic, amphoteric, or nonionic film-forming polymer, also called a setting agent or a setting resin, or the like, and can be any of those conventionally incorporated in hair-styling agents. Specific examples thereof can include the followings:

Acrylic and vinyl setting agent resins:

Anionic: alkyl acrylate/diacetoneacrylamide copolymers (PLASCIZE L-53P, PLASCIZE L-9909B, PLASCIZE L-9948B, etc. (all manufactured by GOO CHEMICAL CO., LTD.)), alkyl acrylate/octylacrylamide copolymers (Dermacryl 79 (manufactured by Nippon NSC Ltd.)), polyethylene glycol/polypropylene glycol-25/dimethicone/acrylate copolymers (LUVIFLEX SILK (manufactured by BASF)), acrylic acid/amide acrylate/ethyl acrylate copolymers (ULTRAHOLD 8 and ULTRAHOLD Strong (manufactured by BASF)), alkyl acrylate copolymers (ANISET NF-1000, ANISET HS-3000, etc. (manufactured by OSAKA ORGANIC CHEMICAL INDUSTRY LTD.)), etc.

Amphoteric: octylamide acrylate/hydroxypropyl acrylate/butylaminoethyl methacrylate copolymers (AMPHOMER SH30 and AMPHOMER LV-71 (manufactured by Nippon NSC Ltd.)), methacryloyloxyethylcarboxybetaine/alkyl methacrylate copolymers (YUKAFORMER R205, YUKAFORMER 301, YUKAFORMER SM, YUKAFORMER 104D, etc. (manufactured by Mitsubishi Chemical Corp.), and RAM Resin-1000, RAM Resin-2000, RAM Resin-3000, and RAM

Resin-4000 (manufactured by OSAKA ORGANIC CHEMICAL INDUSTRY LTD.)), dimethyl diallyl ammonium chloride/acrylic acid copolymers (MERQUAT 280 and MERQUAT 295 (manufactured by Nalco Company)), dimethyl diallyl ammonium chloride/acrylamide/acrylic acid copolymers (MERQUAT Plus 3330 and MERQUAT Plus 3331(manufactured by Nalco Company)), etc.

Cationic: vinylpyrrolidone/dimethylaminoethyl methacrylate copolymer diethyl sulfate (H.C. Polymer 1S (M) and H.C. Polymer 2 (manufactured by OSAKA ORGANIC CHEMICAL INDUSTRY LTD.), and GAFQUAT 755N (manufactured by ISP CORP.)), vinylpyrrolidone/dimethylaminopropylmethacrylamide/lauryl dimethylaminopropylmethacrylamide copolymers (STYLEZE W-20 (manufactured by ISP CORP.)), vinylpyrrolidone/N,N-dimethylaminoethyl methacrylate/alkyl acrylate/tripropylene glycol diacrylate copolymers (COSQUAT GA467 and COSQUAT GA468 (manufactured by OSAKA ORGANIC CHEMICAL INDUSTRY LTD.)), poly(dimethyl methylene piperidinium chloride) (MERQUAT 100 (manufactured by Nalco Company)), dimethyl diallyl ammonium chloride/acrylamide copolymers (MERQUAT 550 (manufactured by Nalco Company)), trimethylaminopropylacrylamide chloride/dimethylacrylamide copolymers, etc.

Nonionic: polyvinylpyrrolidone (LUVISKOL K17, LUVISKOL K30, and LUVISKOL K90 (manufactured by BASF), and PVP K (manufactured by ISP CORP.)), vinylpyrrolidone/vinyl acetate copolymers (PVP/VA S-630, PVP/VA E-735, and PVP/VA E-335 (all manufactured by ISP CORP.), LUVISKOL VA73W and LUVISKOL 37E (all manufactured by BASF), and PVA-6450 (manufactured by OSAKA ORGANIC CHEMICAL INDUSTRY LTD.)), vinylmethyl ether/alkyl maleate copolymers (GANTREZ A-425, GANTREZ ES-225, GANTREZ ES-335, etc. (manufactured by ISP CORP.)), vinylpyrrolidone/methacrylamide/vinylimidazole copolymers (LUVISET Clear (manufactured by BASF)), polyvinylcaprolactam (LUVISKOL Plus (manufactured by BASF)), etc.

Urethane setting agent resin:

IODOSOL PUD (manufactured by Nippon NSC Ltd.), LUVISET P.U.R. (manufactured by BASF), polymers described in JP-A-2006-213706, etc.; and acrylic urethane resins such as DynamX (manufactured by Nippon NSC Ltd.) and polymers described in Japanese Patent Application No. 2006-183144.

Polysaccharide setting agent resin:

Gum arabic, glucan, succinoglycan, carrageenan, karaya gum, tragacanth gum, guar gum, locust bean gum, galactomannan gum, xanthan gum, starch, carob gum, quince seed, casein, dextrin, gelatin, sodium pectate, sodium alginate, methylcellulose, ethylcellulose, carboxymethylcellulose, hydroxyethylcellulose, hydroxypropylcellulose, crystalline cellulose, O-[2-hydroxy-3-(trimethylammonio)propyl]hydroxyethylcellulose chloride, O-[2-hydroxy-3-(trimethylammonio)propyl]guar gum chloride, O-[2-hydroxy-3-(trimethylammonio)propyl]locust bean gum chloride, hydroxypropyl trimonium starch chloride, etc.

The setting agent resin incorporated in the hair cosmetic of the present invention is not particularly limited, and one or two or more of the setting agent resins as listed above can be selected appropriately for use. Acrylic, vinyl, or urethane setting agent resins are particularly preferable.

The amount of the setting agent resin incorporated in the hair cosmetic of the present invention is generally 0.1 to 30% by mass, preferably 0.5 to 20% by mass, more preferably 1 to 15% by mass. If the amount is less than 0.1% by mass, moisture resistance may be insufficient. If the setting agent resin is incorporated in an amount exceeding 30% by mass, the resulting hair cosmetic may make hair bristly.

The hair cosmetic of the present invention comprises an alcohol in addition to the adhesive setting resin and the setting agent resin.

One or two or more selected from alcohols generally used in cosmetics, such as ethanol, can be selected appropriately and used as the alcohol in the hair cosmetic of the present invention. The amount of the alcohol incorporated is not particularly limited and can vary depending on the form of the hair cosmetic. The alcohol is usually incorporated in an amount from the lower limit for use as a solvent in the adhesive setting resin to 80% by mass. Moreover, in some times, it is preferred that the amount of the alcohol incorporated should be adjusted according to the amount of water incorporated, in terms of controlling usability.

The hair cosmetic of the present invention comprises the novel adhesive setting resin and further comprises the setting agent resin. As a result, the hair cosmetic of the present invention exerts the abilities to set and arrange hair and exhibits excellent moisture resistance (ability to retain set hairstyles). Its form can be provided as various forms such as hair liquids, hair foams, hair mousse, hair sprays, hair mists, hair gels, and hair waxes.

The hair cosmetic of the present invention may comprise, for example, other ingredients conventionally used in hair cosmetics such as water according to the form thereof, without impairing the effect of the present invention.

Examples

Hereinafter, the present invention will be described in more detail with reference to specific examples. However, the present invention is not intended to be limited to Examples below. Moreover, the amount of each ingredient incorporated in Examples, etc., below represents % by mass, unless otherwise specified.

(Production Examples and Comparative Production Examples)

Monomers were polymerized according to the composition shown in Table 1 below to prepare adhesive setting resins of the present invention (Production Examples 1 to 6), a monomer C-free resin of Comparative Production Example 1, and a monomer D-free resin of Comparative Production Example 2.
Specifically, a mixture of monomers mixed in the total amount of 100 parts was prepared in advance. 100 parts of ethanol were added to a 1-L five-neck flask equipped with a dropping funnel containing this mixture, a reflux condenser, a thermometer, a tube for nitrogen substitution, and a stirrer. At the point in time when the ethanol was in a reflux state (approximately 80˚C) by heating under nitrogen flow, 1 part of a polymerization initiator (2,2'-azobisisobutyronitrile) was added into this ethanol, and the mixture was continuously added dropwise thereto for 2 hours. Then, the mixture was left for 8 hours in a reflux state to allow polymerization reaction to proceed. Next, the solvent was distilled off from the solution in the five-neck flask, and ethanol was added such that the solvent content in this solution was adjusted to obtain a hair cosmetic base solution having a 50% solid content.
[Table 1]

| Classification | Chemical structure (trade name) | Manufacturer | Production Example 1 | Production Example 2 | Production Example 3 | Production Example 4 | Production Example 5 | Production Example 6 | Comparative Production Example 1 | Comparative Production Example 2 |
|---|---|---|---|---|---|---|---|---|---|---|
| A | Butyl acrylate | Idemitsu Kosan Co., Ltd. | 40 | 35 | 30 | 15 | | | | 40 |
| A | Ethyl acrylate | TOAGOSEI CO., LTD. | | | | | 30 | | 30 | |
| A | Stearyl methacrylate (BLEMMER SMA) | NOF CORP. | | | | | | | | |
| A | Hydroxyethyl acrylate (HEA) | OSAKA ORGANIC CHEMICAL INDUSTRY LTD. | | | | 15 | | | 30 | |
| A | Methoxyethyl acrylate (Acrix C-1) | TOAGOSEI CO., LTD. | | | | | 15 | | | |
| B | Dimethylacrylamide (DMAA) | KOHJIN CHEMICAL CO., LTD. | | 40 | 30 | 30 | | 40 | 35 | 30 |
| B | Dimethylaminopropylacrylamide (DMAPAA) | TOAGOSEI CO., LTD. | | | | | 20 | | | |
| C | Polyoxyethylene glycol acrylate (n=10) (BLEMMER AE-400) | NOF CORP. | 55 | 20 | 15 | 15 | | | | 30 |
| C | Polyoxypropylene glycol acrylate (n=6) (BLEMMER AP-400) | NOF CORP. | | | 20 | 20 | 30 | 55 | | |
| D | Polyoxyethylene glycol diacrylate (n=23) (NK ESTER A-1000) | Shin-Nakamura Chemical Co. | 5 | 5 | 5 | 5 | | | 5 | |
| D | Polyoxyethylene glycol dimethacrylate (n=14) (NK ESTER 14G) | Shin-Nakamura Chemical Co. | | | | | | 5 | | |
| D | Glycerin dimethacrylate (BLEMMER NDMA) | NOF CORP. | | | | | 5 | | | |

(Examples and Comparative Examples)

The resins of Production Examples and Comparative Production Examples were used to prepare samples. The samples were evaluated for the ability to arrange hair, the ability to set hair, the ability to restyle hair, and moisture resistance in use.

Evaluation methods and evaluation criteria for each property are shown below.

1. Ability to arrange hair

0.5 g of the sample was applied to one bundle of black virgin hair (length: 20 cm, mass: 2 g). After drying at room temperature, the hair bundle was evaluated for ease of arrangement in a sensory test by 10 female expert panelists.

<Criteria of evaluation scores>

5: The hair was considerably easily arranged.
4: The hair was slightly easily arranged.
3: Normal.
2: The hair was not much easily arranged.
1: The hair was difficult to arrange.

<Evaluation Criteria>

◎: The total score was 40 or more.
○: The total score was 30 or more and less than 40.
△: The total score was 20 or more and less than 30.
×: The total score was less than 20.

2. Ability to set hair

0.4 g of the sample was applied to black virgin hair (length: 15 cm, weight: 1 g), then spread over the hair using a comb, and styled such that the hair became straight. Five strands were prepared per sample. These strands were dried at 50˚C for 1 hour and then hung on a graduated board. A length (b) of each bent strand was measured in a thermo-hygrostat with a temperature of 30˚C and a humidity of 90% RH. The ability to set hair (ability to keep hairstyles) was determined according to a formula shown below using a length (a) of the bent strand measured in advance before application of the sample. A numeric value closer to 100% represents the higher ability to set hair and more excellent moisture resistance.

$$\text{Ability to keep hairstyles (\%)} = \{(a-b)/a\}\times 100$$

<Evaluation Criteria>

◎: The value was 90% or more.
○: The value was 70% to less than 90%.
Δ: The value was 50% to less than 70%.
x: The value was less than 50%.

3. Ability to restyle hair

0.5 g of the sample was applied to one bundle of black virgin hair (length: 20 cm, mass: 2 g). After drying at room temperature, the hair bundle was evaluated for ease of restyling (ability to restyle hair) in a sensory test by 10 female expert panelists when the hair was styled immediately after the application and restyled one hour thereafter.

<Criteria of evaluation scores>

5: Considerable ability to restyle hair.
4: Slight ability to restyle hair.
3: Normal.
2: Not much ability to restyle hair.
1: No ability to restyle hair.

<Evaluation Criteria>

◎: The total score was 40 or more.
○: The total score was 30 or more and less than 40.
△: The total score was 20 or more and less than 30.
×: The total score was less than 20.

4. Moisture resistance

0.5 g of the sample (hair cosmetic obtained in each Example or Comparative Example) was applied to black virgin hair (length: 20 cm, weight: 2 g). Immediately, the hair was curled using a curler having a curl diameter of 2 cm and dried at 50˚C for 1 hour. The length of a strand of this curled hair was measured and used as an initial value (L0). Next, the dried hair bundle was hung on a graduated board and placed for 3 hours in a thermo-hygrostat with a temperature of 30˚C and a humidity of 90% RH. Then, the length of the hair strand was measured and used as a length (L2) after humidification.
The length of the hair strand is defined as the maximum diameter of curl in a curled state. On the other hand, it is defined as the maximum length from the end on the root side (e.g., the length from the end on the root side to the end on the hair tip side) when the hair is partially or wholly uncurled.
The curl retention value was calculated according to the following formula, and moisture resistance was evaluated according to the evaluation criteria shown below.

$$\text{Curl retention value (\%)} = \{(20-L2)/(20-L0)\} \times 100$$

A curl retention value closer to 100% represents the higher rate of curl retention and more excellent moisture resistance (i.e., ability to retain set hairstyles).

<Evaluation Criteria>

◎: The curl retention value was 90% or more.
○: The curl retention value was 70% or more to less than 90%.
△: The curl retention value was 50% or more to less than 70%.
×: The curl retention value was less than 50%.

(1) In the case of using nonionic resin as setting agent resin

Samples were prepared according to the composition listed in Table 2 below. The samples were prepared by adding each polymer of (3) and/or (4) to a mixed solution of (1) and (2) and stirring the mixture. Subsequently, the properties of each sample were evaluated according to the criteria described above. The results are also shown in Table 2.

[Table 2]

| | Comparative Example 1 | Comparative Example 2 | Example 1 | Example 2 | Example 3 | Example 4 |
|---|---|---|---|---|---|---|
| (1) Water | 60 | 60 | 59.9 | 59 | 55 | 50 |
| (2) Ethanol | 35 | 35 | 35 | 35 | 35 | 35 |

(continued)

| | Comparative Example 1 | Comparative Example 2 | Example 1 | Example 2 | Example 3 | Example 4 |
|---|---|---|---|---|---|---|
| (3) (Vinylpyrrolidone/VA) copolymer | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ○ | Δ | Δ | ◎ | ◎ |
| Ability to set hair | Δ | Δ | Δ | Δ | ○ | ○ |
| Ability to restyle hair | × | ○ | × | Δ | ○ | ◎ |
| Moisture resistance | Δ | Δ | ○ | ○ | ○ | ○ |

As is evident from the results shown in Table 2, the adhesive setting resin (Production Example 3)-free sample (Comparative Example 1) was inferior in the ability to arrange hair and the ability to restyle hair and also had insufficient moisture resistance. Moreover, the samples comprising both the adhesive setting resin and the setting agent resin (Examples 1 to 4) were improved in moisture resistance, compared with the sample free from the (vinylpyrrolidone/VA) copolymer as a setting agent resin (Comparative Example 2).

Samples were prepared according to the composition listed in Table 3 below, and the properties of each sample were evaluated according to the criteria described above. The results are also shown in Table 3.

[Table 3]

| | Comparative Example 1 | Comparative Example 3 | Comparative Example 4 | Example 5 | Example 6 | Example 3 | Example 7 | Example 8 | Example 9 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) (vinylpyrrolidone/VA) copolymer | 5 | 5 | 5 | 5 | 5 | 5 | 5 | 5 | 5 |
| (4) Comparative Production Example 1 | | 5 | | | | | | | |
| (4) Comparative Production Example 2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | Δ | ○ | ○ | Δ | ○ | ○ | ○ | ○ | Δ |

(continued)

| | Comparative Example 1 | Comparative Example 3 | Comparative Example 4 | Example 5 | Example 6 | Example 3 | Example 7 | Example 8 | Example 9 |
|---|---|---|---|---|---|---|---|---|---|
| Ability to restyle hair | × | Δ | Δ | ○ | ○ | ○ | ○ | ○ | ○ |
| Moisture resistance | Δ | ○ | Δ | ○ | ○ | ○ | ○ | ○ | ○ |

The adhesive setting resin (Production Examples 1 to 6)-free sample (Comparative Example 1) and the samples (Comparative Examples 3 and 4) comprising the monomer Cor D-free resin (Comparative Production Example 1 or 2) failed to exert the sufficient abilities to arrange and restyle hair.

(2) In the case of using anionic resin as setting agent resin

Samples were prepared according to the composition listed in Table 4 below. The samples were prepared by adding each polymer of (3) and/or (4) to a mixed solution of (1) and (2) and stirring the mixture. Subsequently, the properties of each sample were evaluated according to the criteria described above. The results are also shown in Table 4.
[Table 4]

| | Comparative Example 5 | Comparative Example 2 | Example 10 | Example 11 | Example 12 | Example 13 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3) (Alkyl acrylate/ diacetoneacrylamide) copolymer | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ○ | Δ | ○ | ◎ | ◎ |
| Ability to set hair | ○ | Δ | ○ | ○ | ○ | ◎ |
| Ability to restyle hair | × | ○ | × | Δ | ○ | ◎ |
| Moisture resistance | ◎ | △ | ◎ | ◎ | ◎ | ◎ |

As is evident from the results shown in Table 4, the adhesive setting resin (Production Example 3)-free sample (Comparative Example 5) was inferior in the ability to arrange hair and the ability to restyle hair. The sample free from the (alkyl acrylate/diacetoneacrylamide) copolymer as a setting agent resin (Comparative Example 2) had insufficient moisture resistance.
Samples were prepared according to the composition listed in Table 5 below, and the properties of each sample were evaluated according to the criteria described above. The results are also shown in Table 5.

[Table 5]

| | Comparative Example 5 | Comparative Example 6 | Comparative Example 7 | Example 14 | Example 15 | Example 12 | Example 16 | Example 17 | Example 18 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) (Alkyl actylate/diacetoneacrylamide) copolymer | 5 | 5 | 5 | 5 | 5 | 5 | 5 | 5 | 5 |
| (4) Comparative Production Example 1 | | 5 | | | | | | | |
| (4) Comparative Production Example 2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Examples 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ○ | ◎ | ◎ | ○ | ○ |

(continued)

| | Comparative Example 5 | Comparative Example 6 | Comparative Example 7 | Example 14 | Example 15 | Example 12 | Example 16 | Example 17 | Example 18 |
|---|---|---|---|---|---|---|---|---|---|
| Ability to set hair | ○ | ○ | ○ | ○ | ○ | ○ | ○ | ○ | ○ |
| Ability to restyle hair | × | Δ | Δ | ○ | ○ | ○ | ○ | ○ | ○ |
| Moisture resistance | ◎ | ◎ | ◎ | ◎ | ◎ | ◎ | ◎ | ◎ | ◎ |

The adhesive setting resin (Production Examples 1 to 6)-free sample (Comparative Example 5) and the samples (Comparative Examples 6 and 7) comprising the monomer Cor D-free resin (Comparative Production Example 1 or 2) failed to exert the sufficient abilities to arrange and restyle hair.

(3) In the case of using cationic resin as setting agent resin

Samples were prepared according to the composition listed in Table 6 below. The samples were prepared by adding each polymer of (3) and/or (4) to a mixed solution of (1) and (2) and stirring the mixture. Subsequently, the properties of each sample were evaluated according to the criteria described above. The results are also shown in Table 6.
[Table 6]

| | Comparative Example 8 | Comparative Example 2 | Example 19 | Example 20 | Example 21 | Example 22 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3) Polyquaternium-11 | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ○ | Δ | ○ | ◎ | ◎ |
| Ability to set hair | ○ | Δ | ○ | ○ | ○ | ◎ |
| Ability to restyle hair | × | ○ | × | Δ | ○ | ◎ |
| Moisture resistance | ○ | Δ | ○ | ○ | ○ | ○ |

As is evident from the results shown in Table 6, the adhesive setting resin (Production Example 3)-free sample (Comparative Example 8) was inferior in the ability to arrange hair and the ability to restyle hair. The sample free from polyquaternium-11 as a setting agent resin (Comparative Example 2) had insufficient moisture resistance.
Samples were prepared according to the composition listed in Table 7 below, and the properties of each sample were evaluated according to the criteria described above. The results are also shown in Table 7.

[Table 7]

| | Comparative Example 8 | Comparative Example 9 | Comparative Example 10 | Example 23 | Example 24 | Example 21 | Example 25 | Example 26 | Example 27 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) Polyquaternium-11 | 5 | 5 | 5 | 5 | 5 | 5 | 5 | 5 | 5 |
| (4) Comparative Production Example 1 | | 5 | | | | | | | |
| (4) Comparative Production Example 2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | ○ | ○ | ○ | ○ | ○ | ○ | ○ | ○ | ○ |

(continued)

| | Comparative Example 8 | Comparative Example 9 | Comparative Example 10 | Example 23 | Example 24 | Example 21 | Example 25 | Example 26 | Example 27 |
|---|---|---|---|---|---|---|---|---|---|
| Ability to restyle hair | × | Δ | Δ | ○ | ○ | ○ | ○ | ○ | ○ |
| Moisture resistance | ○ | ○ | ○ | ○ | ○ | ○ | ○ | ○ | ○ |

The adhesive setting resin (Production Examples 1 to 6)-free sample (Comparative Example 8) and the samples (Comparative Examples 9 and 10) comprising the monomer Cor D-free resin (Comparative Production Example 1 or 2) failed to exert the sufficient abilities to arrange and restyle hair.

(4) In the case of using amphoteric resin as setting agent resin

Samples were prepared according to the composition listed in Table 8 below. The samples were prepared by adding each polymer of (3) and/or (4) to a mixed solution of (1) and (2) and stirring the mixture. Subsequently, the properties of each sample were evaluated according to the criteria described above. The results are also shown in Table 8.

[Table 8]

| | Comparative Example 11 | Comparative Example 2 | Example 28 | Example 29 | Example 30 | Example 31 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3) (Methacryloyloxyethylcarboxybetaine/alkyl methacrylate) copolymer | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ○ | Δ | Δ | ◎ | ◎ |
| Ability to set hair | ○ | Δ | ○ | ○ | ○ | ○ |
| Ability to restyle hair | × | ○ | × | Δ | ○ | ◎ |
| Moisture resistance | Δ | Δ | ○ | ○ | ○ | ○ |

As is evident from the results shown in Table 8, the adhesive setting resin (Production Example 3)-free sample (Comparative Example 11) was inferior in the ability to arrange hair and the ability to restyle hair. The sample free from the (methacryloyloxyethylcarboxybetaine/alkyl methacrylate) copolymer as a setting agent resin (Comparative Example 2) had insufficient moisture resistance.

Samples were prepared according to the composition listed in Table 9 below, and the properties of each sample were evaluated according to the criteria described above. The results are also shown in Table 9.

[Table 9]

| | Comparative Example 11 | Comparative Example 12 | Comparative Example 13 | Example 32 | Example 33 | Example 30 | Example 34 | Example 35 | Example 36 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) (Methacryloyloxyethylcarboxy betaine/alkyl methacrylate) copolymer | 5 | 5 | 5 | 5 | 5 | 5 | 5 | 5 | 5 |
| (4) Comparative Production Example 1 | | 5 | | | | | | | |
| (4) Comparative Production Example 2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | x | Δ | Δ | ○ | ◎ | ◎ | ◎ | ○ | ○ |
| Ability to set hair | ○ | ○ | ○ | ○ | ○ | ○ | ○ | ○ | ○ |
| Ability to restyle hair | × | Δ | Δ | ○ | ○ | ○ | ○ | ○ | ○ |
| Moisture resistance | Δ | ○ | ○ | ○ | ○ | ○ | ○ | ○ | ○ |

The adhesive setting resin (Production Examples 1 to 6)-free sample (Comparative Example 11) and the samples (Comparative Examples 12 and 13) comprising the monomer C- or D-free resin (Comparative Production Example 1 or 2) failed to exert the sufficient abilities to arrange and restyle hair.

(5) In the case of urethane resin as setting agent resin

Samples were prepared according to the composition listed in Table 10 below. The samples were prepared by adding each polymer of (3) and/or (4) to a mixed solution of (1) and (2) and stirring the mixture. Subsequently, the properties of each sample were evaluated according to the criteria described above. The results are also shown in Table 10.

[Table 10]

| | Comparative Example 14 | Comparative Example 2 | Example 37 | Example 38 | Example 39 | Example 40 |
|---|---|---|---|---|---|---|
| (1) Water | 60 | 60 | 59.9 | 59 | 55 | 50 |
| (2) Ethanol | 35 | 35 | 35 | 35 | 35 | 35 |
| (3) Silylated urethane resin*) | 5 | | 5 | 5 | 5 | 5 |
| (4) Production Example 3 | | 5 | 0.1 | 1 | 5 | 10 |
| Total | 100 | 100 | 100 | 100 | 100 | 100 |
| | | | | | | |
| Ability to arrange hair | × | ○ | Δ | ○ | ◎ | ◎ |
| Ability to set hair | ◎ | Δ | ◎ | ◎ | ◎ | ◎ |
| Ability to restyle hair | × | ○ | × | Δ | ○ | ◎ |
| Moisture resistance | ◎ | Δ | ◎ | ◎ | ◎ | ◎ |
| *) Silylated urethane resin described as Production Example in JP-A-2006-213706. | | | | | | |

As is evident from the results shown in Table 10, the adhesive setting resin (Production Example 3)-free sample (Comparative Example 14) was inferior in the ability to arrange hair and the ability to restyle hair. The sample free from the silylated urethane resin as a setting agent resin (Comparative Example 2) had insufficient moisture resistance.

Samples were prepared according to the composition listed in Table 11 below, and the properties of each sample were evaluated according to the criteria described above. The results are also shown in Table 11.

[Table 11]

| | Comparative Example 14 | Comparative Example 15 | Comparative Example 16 | Example 41 | Example 42 | Example 39 | Example 43 | Example 44 | Example 45 |
|---|---|---|---|---|---|---|---|---|---|
| (1) Water | 60 | 55 | 55 | 55 | 55 | 55 | 55 | 55 | 55 |
| (2) Ethanol | 35 | 35 | 35 | 35 | 35 | 35 | 35 | 35 | 35 |
| (3) Silylated urethane resin*) | 5 | 5 | 5 | 5 | 5 | 5 | 5 | 5 | 5 |
| (4) Comparative Production Example 1 | | 5 | | | | | | | |
| (4) Comparative Production Example 2 | | | 5 | | | | | | |
| (4) Production Example 1 | | | | 5 | | | | | |
| (4) Production Example 2 | | | | | 5 | | | | |
| (4) Production Example 3 | | | | | | 5 | | | |
| (4) Production Example 4 | | | | | | | 5 | | |
| (4) Production Example 5 | | | | | | | | 5 | |
| (4) Production Example 6 | | | | | | | | | 5 |
| Total | 100 | 100 | 100 | 100 | 100 | 100 | 100 | 100 | 100 |
| | | | | | | | | | |
| Ability to arrange hair | × | Δ | Δ | ○ | ◎ | ◎ | ◎ | ○ | ○ |
| Ability to set hair | ◎ | ◎ | ◎ | ◎ | ◎ | ◎ | ◎ | ◎ | ◎ |

(continued)

| | Comparative Example 14 | Comparative Example 15 | Comparative Example 16 | Example 41 | Example 42 | Example 39 | Example 43 | Example 44 | Example 45 |
|---|---|---|---|---|---|---|---|---|---|
| Ability to restyle hair | × | Δ | Δ | ○ | ○ | ○ | ○ | ○ | ○ |
| Moisture resistance | ◎ | ◎ | ◎ | ◎ | ◎ | ◎ | ◎ | ◎ | ◎ |
| *) Silylated urethane resin described as Production Example in JP-A-2006-213706. | | | | | | | | | |

The sample (Comparative Example 14) comprising no adhesive setting resin (Production Examples 1 to 6) and the samples (Comparative Examples 15 and 16) comprising the monomer C- or D-free resin (Comparative Production Example 1 or 2) failed to exert the sufficient abilities to arrange and restyle hair.

The other Examples are shown below.

(Example 51)

Liquid styling agent

| | | |
|---|---|---|
| (1) | ion-exchanged water | balance |
| (2) | PEG-6 | 5 |
| (3) | PEG-8 | 5 |
| (4) | PEG-32 | 5 |
| (5) | sorbitol | 3 |
| (6) | ethanol | 35 |
| (7) | fragrance | q.s. |
| (8) | polymer obtained in Production Example 3 | 5 |
| (9) | (alkyl acrylate/diacetone acrylamide) copolymer | 2.5 |
| (10) | citric acid | q.s. |

<Production Process>

Water-soluble ingredients (2) to (5) were added to water (1) and dissolved by stirring to prepare aqueous parts. Next, (7) was added to (6), and the mixture was stirred for solubilization. Then, (8) and (9) were added thereto, and the mixture was stirred to prepare alcohol parts. The aqueous parts and the alcohol parts were mixed, and (10) was added to the mixture to obtain a liquid styling agent.

(Example 52)

Hair-styling gel

| | | |
|---|---|---|
| (2) | carboxyvinyl polymer | 0.7 |
| (2) | polymer obtained in Production Example 3 | 5.0 |
| (3) | glycerin | 2.5 |
| (4) | 1,3-butylene glycol | 2.5 |
| (5) | polyoxyethylene octyldodecyl ether (20EO) | 0.5 |
| (6) | polyether-modified dimethylpolysiloxane | 1.0 |
| (7) | sodium hydroxide (adjusted to pH 7.5) | q.s. |
| (8) | ethanol | 20.0 |
| (9) | polyoxyethylene octyldodecyl ether | 0.1 |
| (10) | fragrance | 0.1 |
| (11) | trisodium edetate | 0.03 |
| (12) | ion-exchanged water | balance |
| (13) | (alkyl acrylate/diacetone acrylamide) copolymer | 5.0 |

<Production Process>

(6) was added to (3), (4), (5), and a portion of (12), and the mixture was emulsified using a homo mixer. Subsequently, a portion of the remaining (12) was added to the emulsion to prepare emulsified parts. On the other hand, (1), (2), (7), (8), (9), (10), (11), and (13) were uniformly dissolved in the remaining portion of (12). To this solution, the

emulsified parts were added to obtain a hair-styling gel emulsion.

(Example 53)

Hair-styling gel

| | | |
|---|---|---|
| (1) | Hydroxyethylcellulose | 0.7 |
| (2) | Polymer obtained in Production Example 3 | 12.0 |
| (3) | Propylene glycol | 2.5 |
| (4) | 1,3-Butylene glycol | 2.5 |
| (5) | Polyoxyethylene hydrogenated castor oil (40EO) | 0.5 |
| (6) | Amino-modified high-molecular-weight silicone | 1.0 |
| (7) | Sodium hydroxide (adjusted to pH 7.5) | q.s. |
| (8) | Ethanol | 20.0 |
| (9) | Polyoxyethylene octyldodecyl ether | 0.1 |
| (10) | Fragrance | 0.1 |
| (11) | Trisodium edetate | 0.03 |
| (12) | Ion-exchanged water | balance |
| (13) | (Octylacrylamide/hydroxypropyl acrylate/butylaminoethyl methacrylate) copolymer | 16.0 |

<Production Process>

A hair-styling gel was produced according to Example 52.

(Example 54)

Hair-styling gel

| | | |
|---|---|---|
| (1) | (PEG-240/decyltetradeceth-20/HDI) copolymer | 2.0 |
| (2) | polymer obtained in Production Example 3 | 1.0 |
| (3) | diglycerin | 2.5 |
| (4) | polyethylene glycol 1000 | 2.5 |
| (5) | polyoxyethylene hydrogenated castor oil (40EO) | 0.5 |
| (6) | dimethylpolysiloxane modified with hydroxy at both ends (1,000,000 mPa-s) | 1.0 |
| (7) | sodium hydroxide (adjusted to pH 7.5) | q.s. |
| (8) | ethanol | 20.0 |
| (9) | polyoxyethylene octyldodecyl ether | 0.1 |
| (10) | fragrance | 0.1 |
| (11) | trisodium edetate | 0.03 |
| (12) | ion-exchanged water | balance |
| (13) | (alkyl acrylate/diacetone acrylamide) copolymer | 1.0 |

<Production Process>

A hair-styling gel was produced according to Example 52.

(Example 55)

Hair-styling gel

| | | |
|---|---|---|
| (1) | Polymer obtained in Production Example 3 | 1.0 |
| (2) | Silylated urethane resin (Production Example of 2006-213706) JP-A- | 2.5 |
| (3) | Glycerin | 2.5 |
| (4) | Hydroxyethyl urea | 2.5 |
| (5) | Polyoxyethylene hydrogenated castor oil (40EO) | 0.5 |
| (6) | Dimethylpolysiloxane (1,000 mPa·s) | 1.0 |
| (7) | Triethanolamine (adjusted to pH 7.5) | q.s. |
| (8) | Ethanol | 20.0 |
| (9) | Polyoxyethylene octyldodecyl ether | 0.1 |
| (10) | Fragrance | 0.1 |
| (11) | Trisodium edetate | 0.03 |
| (12) | Ion-exchanged water | balance |
| (13) | (Octylacrylamide/hydroxypropyl acrylate/butylaminoethyl methacrylate) copolymer | 1.0 |

<Production Process>

A hair-styling gel was produced according to Example 52.

(Example 56)

Styling mousse

| | | |
|---|---|---|
| (1) | dimethylpolysiloxane (20 mPa·s) | 5.0 |
| (2) | isoparaffin | 5.0 |
| (3) | high-molecular-weight polysiloxane | 2.0 |
| (4) | amino-modified high-molecular-weight silicone | 0.5 |
| (5) | 1,3-butylene glycol | 3.0 |
| (6) | polyoxyethylene hydrogenated castor oil (40EO) | 2.0 |
| (7) | polymer obtained in Production Example 3 | 10.0 |
| (8) | polyoxyethylene/polyoxypropylene decyl ether (12EO·2PO) | 1.0 |
| (9) | behenyl trimethyl ammonium chloride | 0.1 |
| (10) | phenoxyethanol | 0.1 |
| (11) | ethanol | 8.0 |
| (12) | ion-exchanged water | balance |
| (13) | fragrance | q.s. |
| (14) | (octylacrylamide/hydroxypropyl acrylate/butylaminoethyl methacrylate) copolymer | 1.0 |

<Production Process>

(3) and (4) were dissolved in (1) and (2) by stirring, and this solution was added to (5), (6), and a portion of (12). The mixture was emulsified using a homo mixer (emulsified parts). On the other hand, (7) was added to the remaining portion of (12) (aqueous-phase parts). (8), (9), (10), (13), and (14) were added to (11) and dissolved by stirring. This solution was added to the aqueous-phase parts, to which the emulsified parts were further added and uniformly mixed to prepare a stock solution. 90 parts of this stock solution were charged into a can for aerosol. The can was valved, and 10 parts of liquefied petroleum gas (LPG) were charged thereto to obtain a styling mousse.

(Example 57)

Styling mousse

| | | |
|---|---|---|
| (1) | Dimethylpolysiloxane (20 mPa·s) | 5.0 |
| (2) | High-molecular-weight methylpolysiloxane modified with hydroxy at both ends | 5.0 |
| (3) | (Octylacrylamide/hydroxypropyl acrylate/butylaminoethyl methacrylate) copolymer | 1.0 |
| (4) | 1,3-Butylene glycol | 3.0 |
| (5) | Polyoxyethylene hydrogenated castor oil (40EO) | 2.0 |
| (6) | Polymer obtained in Production Example 3 | 0.0 |
| (7) | Polyoxyethylene cetyl ether (20EO) | 0.5 |
| (8) | Stearyl trimethyl ammonium chloride | 0.1 |
| (9) | Phenoxyethanol | 0.5 |
| (10) | Ethanol | 8.0 |
| (11) | Ion-exchanged water | balance |
| (12) | Fragrance | q.s. |

<Production Process>

Styling mousse was produced according to Example 56.

(Example 58)

Styling mousse

| | | |
|---|---|---|
| (1) | dimethylpolysiloxane (6 mPa·s) | 5.0 |
| (2) | isoparaffin | 5.0 |
| (3) | (PEG/amodimethicone) copolymer | 1.0 |
| (4) | 1,3-butylene glycol | 3.0 |
| (5) | polyoxyethylene hydrogenated castor oil (40EO) | 2.0 |
| (6) | polymer obtained in Production Example 3 | 1.0 |
| (7) | lauric acid diethanolamide | 0.2 |
| (8) | stearoxy hydroxypropylamine | 0.1 |
| (9) | phenoxyethanol | 0.1 |
| (10) | ethanol | 8.0 |
| (11) | ion-exchanged water | balance |
| (12) | fragrance | q.s. |
| (13) | (octylacrylamide/hydroxypropyl acrylate/butylaminoethyl methacrylate) copolymer | 0.5 |

<Production Process>

A styling mousse was produced according to Example 56.

(Example 59)

Styling mousse

| | | |
|---|---|---|
| (1) | Dimethylpolysiloxane (20 mPa·s) | 5.0 |

(continued)

| | | |
|---|---|---|
| (2) | Volatile isoparaffin | 5.0 |
| (3) | Polyether-modified high-molecular-weight silicone | 3.5 |
| (4) | 1,3-Butylene glycol | 3.0 |
| (5) | Polyoxyethylene hydrogenated castor oil (40EO) | 2.0 |
| (6) | Silylated urethane resin (Production Example of JP-A- 2006-213706) | 10.0 |
| (7) | Polyoxyethylene/polyoxypropylene behenyl ether (12EO-1PO) | 1.0 |
| (8) | Cationized trehalose | 0.1 |
| (9) | Phenoxyethanol | 0.1 |
| (10) | Ethanol | 8.0 |
| (11) | Ion-exchanged water | balance |
| (12) | Fragrance | q.s. |
| (13) | Polymer obtained in Production Example 3 | 3.0 |

<Production Process>

Styling mousse was produced according to Example 56.

(Example 60)

Styling spray

| | | |
|---|---|---|
| (1) | Polymer obtained in Production Example 3 | 5.0 |
| (2) | Pyrocarboxylic acid-modified dimethylpolysiloxane | 0.5 |
| (3) | Alcohol | balance |
| (4) | Fragrance | q.s. |
| (5) | (Alkyl acrylate/diacetoneacrylamide) copolymer | 10.0 |

<Production Process>

(1) to (5) were mixed and dissolved to prepare a stock solution. 50% of this stock solution and 50% of dimethyl ether were charged to an aerosol can to prepare an aerosol spray.

(Example 61)

Styling spray

A stock solution was prepared in the same way as in Example 15. 99.33% of the stock solution and 0.67% of nitrogen gas were mixed and charged to an aerosol can to prepare an aerosol spray without the use of combustible gas.

(Example 62)

Hair wax

| | | |
|---|---|---|
| (1) | kaolin | 1.0 |
| (2) | volatile isoparaffin | 5.0 |
| (3) | cetyl octanoate | 5.0 |
| (4) | phenylmethylpolysiloxane | 0.5 |
| (5) | candelilla wax | 3.0 |
| (6) | paraffin wax | 8.0 |

(continued)

| | | |
|---|---|---|
| (7) | propylene glycol | 5.0 |
| (8) | glyceryl stearate | 1.0 |
| (9) | polyoxyethylene glycerin monostearate (5EO) | 1.0 |
| (10) | isostearic acid | 1.0 |
| (11) | carboxyvinyl polymer | 0.4 |
| (12) | potassium hydroxide (adjusted to pH 7.5) | q.s. |
| (13) | ion-exchanged water | balance |
| (14) | polymer obtained in Production Example 3 | 5.0 |
| (15) | sorbitol | 3.0 |
| (16) | EDTA-2Na·2$H_2O$ | 0.05 |
| (17) | phenoxyethanol | 0.5 |
| (18) | fragrance | q.s. |
| (19) | highly polymerized polyethylene glycol | 0.1 |
| (20) | Silylated urethane resin (Production Example of JP-A-2006-213706) | 1.0 |

<Production Process>

(16), (7), and (15) were added to (13) and dissolved. Then, (11) was added to the solution and uniformly dispersed by stirring. (1) was added thereto and uniformly dispersed using Disper. The mixture was heated to 85˚C, and a mixture of (2) to (9) dissolved by stirring at 85˚C in the same way as above was then added thereto and uniformly stirred. Then, (12) was added thereto, and the mixture was emulsified using a homo mixer. (14), (15), and (17) to (20) were sequentially added thereto, and the mixture was cooled to 25˚C to obtain a hair wax.

## Claims

**1.** A hair cosmetic comprising an adhesive setting resin, a setting agent resin, and an alcohol, wherein the adhesive setting resin being obtained by polymerizing: at least one monomer represented by the following formula (A) :

[Formula 1]

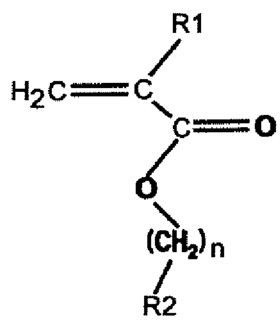

(A)

wherein R1 represents H or $CH_3$; n represents an integer of 0 to 30; $(CH_2)_n$ contains a branched chain; and R2 represents H, OH, $OCH_3$, $OCH_2CH_3$, or phenyl,
and/or
at least one monomer represented by the following formula (B) :

[Formula 2]

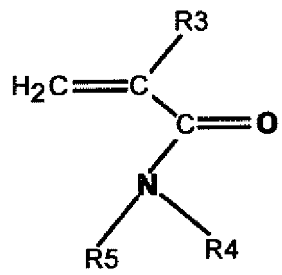

(B)

wherein R3 represents H or $CH_3$; R4 and R5, which may be the same or different, each represent H or $(CH_2)_1R'$; 1 represents an integer of 1 to 3; R' represents H, OH, or -NR"R'"; and R" and R'", which may be the same or different, each represent H or a C1-C3 alkyl group,
and
at least one monomer represented by the following formula (C) :

[Formula 3]

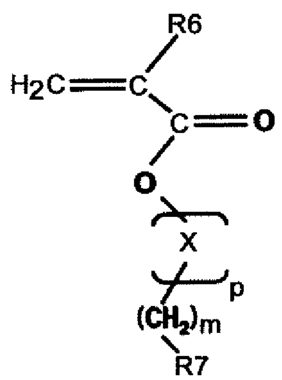

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or glyceryl,
and
at least one monomer represented by the following formula D:

[Formula 4]

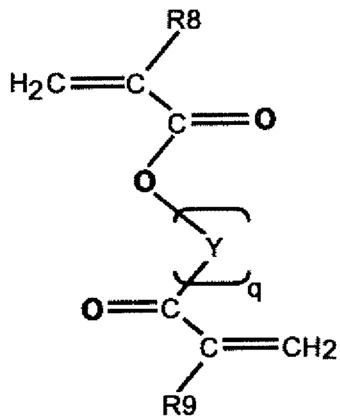

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or a glyceryl group, provided that q represents 1 when Y represents a linear or branched oxyalkylene group having 5 or more carbon atoms.

2. The hair cosmetic according to claim 1, wherein the adhesive setting resin has a structure represented by the following formula (I):

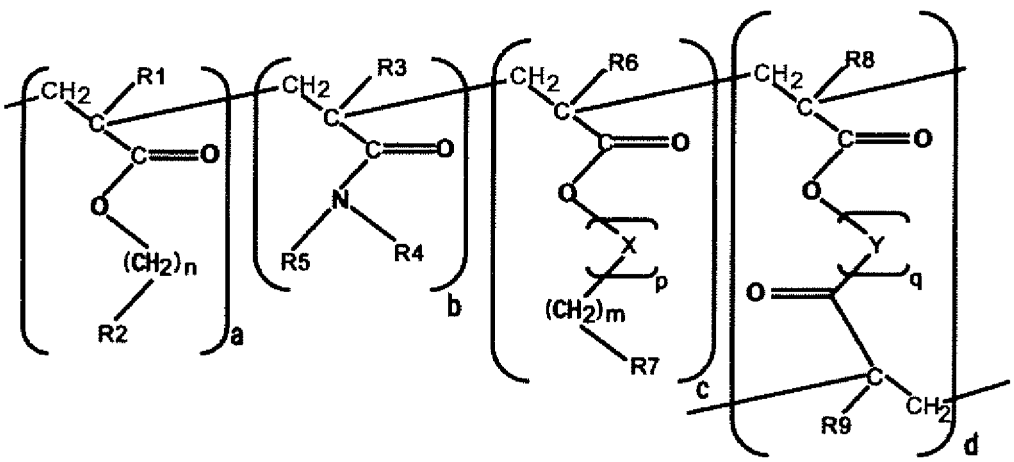

(I)

wherein R1 to R9, n, m, p, and q are as defined in the formulas A to D; a represents a number within the range of $40 < a < 400$; b represents a number within the range of $80 \leq b < 300$; c represents a number within the range of $30 < c < 300$; and d represents a number within the range of $0 < d < 10$.

3. The hair cosmetic according to claim 1 or 2, wherein the setting agent resin is one or two or more selected from anionic, cationic, amphoteric, and nonionic film-forming polymers.

## INTERNATIONAL SEARCH REPORT

International application No.
PCT/JP2010/056057

A. CLASSIFICATION OF SUBJECT MATTER
*A61K8/81*(2006.01)i, *A61K8/34*(2006.01)i, *A61K8/91*(2006.01)i, *A61Q5/06* (2006.01)i

According to International Patent Classification (IPC) or to both national classification and IPC

B. FIELDS SEARCHED

Minimum documentation searched (classification system followed by classification symbols)
A61K8/81, A61K8/34, A61K8/91, A61Q5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 6-316510 A (Osaka Organic Chemical Ind. Co., Ltd.), 15 November 1994 (15.11.1994), claims; paragraph [0042]; each example; example of prescription; paragraph [0131] (Family: none) | 1-3 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
07 May, 2010 (07.05.10)

Date of mailing of the international search report
18 May, 2010 (18.05.10)

Name and mailing address of the ISA/
Japanese Patent Office

Authorized officer

Facsimile No.

Telephone No.

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006213706 A
- JP 2003171244 A
- JP HEI1045546 A
- JP 2006183144 A


### Non-patent literature cited in the description


- Functions of Hair-Styling Agents and State-of-the-Art Technology. Development of Advanced Cosmetics II. CMC Publishing Co., Ltd, 1996